# EUROPÄISCHE PATENTSCHRIFT

(11) **EP 1 629 785 B1**
(45) Veröffentlichungstag und Bekanntmachung des Hinweises auf die Patenterteilung: **22.07.2009**
(21) Anmeldenummer: 05017801.1
(22) Anmeldetag: 17.08.2005
(51) Int. Cl.: A61B 17/28

(54) **Medizinische Zange**
Medical forceps
Pince médicale

(30) Priorität: 24.08.2004 DE 102004041515
(43) Veröffentlichungstag der Anmeldung: 01.03.2006
(73) Patentinhaber: Karl Storz GmbH & Co. KG, 78532 Tuttlingen (DE)
(72) Erfinder: Ganter, Hans, 78532 Tuttlingen (DE)
(74) Vertreter: Heuckeroth, Volker

(56) Entgegenhaltungen:
- EP-A- 0 571 057
- DE-U1- 8 910 462
- US-A- 2 305 156
- US-A- 5 673 841
- US-A- 6 077 290
- US-A1- 2004 167 569

## Beschreibung

Die Erfindung betrifft eine medizinische Zange wie in den Ansprüchen definiert.

Eine medizinische Zange ist aus dem Dokument US-A-6 077 290 bekannt.

Eine medizinische Zange der eingangs genannten Art wird insbesondere im Rahmen der minimal-invasiven Chirurgie für Eingriffe am menschlichen oder tierischen Körper verwendet. Ohne Beschränkung der Allgemeinheit kann die medizinische Zange im Sinne der vorliegenden Erfindung als Fasszange zum Fassen von Gewebe oder als Präparierzange zum Schneiden bzw. Präparieren von Gewebe ausgebildet sein.

Eine aus dem Dokument DE 38 02 651 C2 bekannte medizinische zange weist eine Handhabe auf, die ein bewegliches Griffteil und ein unbewegliches Griffteil aufweist. Das bewegliche Griffteil ist um eine feststehende Schwenkachse, die am handhabungsabgewandten Ende des beweglichen Griffteils angeordnet ist, verschwenkbar in einer Ausnehmung eines unbeweglichen Gehäuseteils der Handhabe befestigt, das seinerseits einstückig mit dem unbeweglichen Griffteil verbunden ist. Das unbewegliche und das bewegliche Griffteil stehen im Wesentlichen quer zur Längsachse des Schafts dieser Zange ab und bilden eine scherengriffartige Anordnung. Im Schaft verläuft in Richtung der Längsachse des Schafts ein Kraftübertragungselement, das axial in Richtung der Längsachse des Schafts beweglich ist. Das Kraftübertragungselement, das in Form eines dünnen Stabes ausgebildet ist, dient der Kraftübertragung vom beweglichen Griffteil auf das bewegliche Werkzeug, beispielsweise ein Maulteil, am distalen Ende des Schafts. Entsprechend steht das Kraftübertragungselement mit dem beweglichen Werkzeug einerseits und dem beweglichen Griffteil andererseits in Wirkverbindung. Bezüglich des beweglichen Griffteils wird diese Wirkverbindung über ein gelenkiges Zwischenstück hergestellt, das seinerseits um eine feststehende Schwenkachse in der Ausnehmung des Gehäuseteils verschwenkbar befestigt ist, und mit dem das Kraftübertragungselement an einem Anlenkungspunkt im Abstand von der vorstehend genannten feststehenden Schwenkachse angelenkt ist. Das gelenkige Zwischenstück ist über einen Stift, der in ein Langloch eingreift, das am beweglichen Griffteil im Abstand zu dessen feststehender Schwenkachse angeordnet ist, gelenkig mit dem beweglichen Griffteil verbunden.

Im praktischen Gebrauch dieser medizinischen Zange hat sich herausgestellt, dass der Gang der Kraftübertragung vom beweglichen Griffteil auf das bewegliche Werkzeug trotz Beachtung geringster Toleranzen und sorgfältigster Fertigung nicht spielfrei ist. Dies hat den Nachteil, dass das bewegliche Werkzeug nicht exakt den Bewegungen des beweglichen Griffteils folgt. Ein weiterer Nachteil dieser bekannten medizinischen Zange besteht darin, dass der Anlenkungspunkt des axial beweglichen Kraftübertragungselements an dem gelenkigen Zwischenstück beim Betätigen des beweglichen Griffteils eine Kreisbewegung ausführt, was eine zusätzliche Bewegung des Kraftübertragungselements quer zur Längsrichtung des Schafts bedingt. Dies ist mitunter ein Grund dafür, dass diese Art von Kraftübertragungsmechanismus nicht vollständig spielfrei arbeitet.

Die aus dem eingangs genannten Dokument US-A-6 077 290 bekannte Zange weist eine Kraftübertragungsmechanik auf, die ein Kraftübertragungselement aufweist, das mit einem linear passgenau in einem Gleitlager geführten Schieber verbunden ist, der in Richtung des Kraftübertragungselements axial beweglich ist, wobei der Schieber über einen Gelenkhebel mit dem beweglichen Griffteil verbunden ist, der mit einem Ende am Schieber und mit dem anderen Ende am beweglichen Griffteil angelenkt ist. Der Schieber ist koaxial mit dem Kraftübertragungselement ausgerichtet.

Aus dem Dokument DE 89 10 462 U1 ist eine medizinische Zange bekannt, bei der das Kraftübertragungselement direkt über einen Gelenkhebel mit dem beweglichen Griffteil verbunden ist.

In dem Dokument EP-A-0 571 057 ist eine medizinische Zange in Form eines Nadelhalter beschrieben, der eine Handhabe mit zwei beweglichen Griffteilen aufweist, die zusammen die Form eines Stabgriffes bilden. Zwischen den beiden beweglichen Griffteilen ist ein Schieber angeordnet, der über eine Kraftbegrenzungsmechanik mit dem Kraftübertragungselement verbunden ist.

In US-A-5 673 841 ist ein medizinisches Instrument offenbart, bei dem das bewegliche Griffteil über einen Gelenkhebel mit dem Kraftübertragungselement verbunden ist. Mit dem Schaft des Instruments ist ein weiterer Hebel schwenkbar an einer Schwenkachse verbunden. Der Hebel ist verschwenkbar mit einem Gestänge verbunden, das wiederum mit einer Hebelstange verschwenkbar verbunden ist. Die Hebelstange ist über einen sich quer zum Kraftübertragungselement erstreckenden Mitnehmer relativ zum Kraftübertragungselement axial verschiebbar mit letzterem verbunden.

Eine hinsichtlich der Kraftübertragungsmechanik vom beweglichen Griffteil auf das bewegliche Werkzeug besonders einfach ausgestaltete medizinische Zange ist aus dem Dokument DE 32 15 949 A1 bekannt.

Bei dieser bekannten medizinischen Zange ist das bewegliche Griffteil am unbeweglichen Griffteil um eine feststehende Schwenkachse verschwenkbar befestigt und weist einen vom handhabungsseitigen Ende des beweglichen Griffteils aus gesehen über die Schwenkachse hinausragenden Fortsatz auf, an dem das Kraftübertragungselement unmittelbar angelenkt ist. Da dieser Fortsatz beim Verschwenken des beweglichen Griffteils eine Kreisbewegung ausführt, besteht auch hier wiederum der Nachteil, dass das axial bewegliche Kraftübertragungselement eine Kreisbewegung ausführt.

Schließlich offenbart das Dokument DE-AS 1 055 751 einen Handgriff für chirurgische Instrumente, der ein feststehendes Griffteil und ein bewegliches Griffteil aufweist, die im Unterschied zu den beiden zuvor genannten bekannten medizinischen Zangen im Wesentlichen in axialer Verlängerung des Schafts verlaufen und eine Griffanordnung bilden, die einem Zangengriff gleicht. Das bewegliche Griffteil ist mit einem in Längsrichtung des Kraftübertragungselements axial beweglichen Schieber durch einen Verbindungsstift unmittelbar gelenkig verbunden. Der Schieber nimmt den Instrumentenaufsatz auf. Als gelenkige Verbindung des beweglichen Griffteils mit dem unbeweglichen Griffteil ist zwischen beiden eine Blattfeder geschaltet, die zwischen dem beweglichen Griffteil und dem festen Griffteil als Hebelarm dient. Durch die unmittelbare Anlenkung des beweglichen Griffteils an dem Schieber führt dieser ebenfalls keine streng lineare Bewegung aus, weil der Anlenkungspunkt des beweglichen Griffteils am Schieber auf Grund der Zwischenschaltung der Blattfeder eine gekrümmte Bewegungskurve durchläuft. Dies hat als Nachteil zur Folge, dass der Hub des Schiebers auf einen geringen Bereich eingeschränkt sein muss, um die Kreisbewegung des Anlenkungspunktes des beweglichen Griffteils am Schieber auf kleine Winkel zu begrenzen. Somit ist auch diese Kraftübertragungsmechanik nicht spielfrei. Ein weiterer Nachteil dieser Kraftübertragungsmechanik besteht darin, dass sie sich nur für Griffteilanordnungen eignet, bei denen die Griffteile im Wesentlichen in geradliniger Verlängerung des Schafts angeordnet sind.

Der Erfindung liegt die Aufgabe zugrunde, eine medizinische Zange der eingangs genannten Art dahingehend weiterzubilden, dass die Kraftübertragungsmechanik vom beweglichen Griffteil auf das bewegliche Werkzeug möglichst spielfrei ist.

Erfindungsgemäß wird diese Aufgabe hinsichtlich der eingangs genannten medizinischen Zange dadurch gelöst, dass der Schieber in Bezug auf das Kraftübertragungselement quer zur Längsachse des Schafts in Richtung zum beweglichen Griffteil hin parallel versetzt angeordnet ist und einen seitlich abstehenden Mitnehmer aufweist, an dem das Kraftübertragungselement festgelegt ist, wobei der Gang der Kraftübertragungsmechanik vom beweglichen Griffteil auf das bewegliche Werkzeug spielfrei ist.

Erfindungsgemäß ist die Kraftübertragungsmechanik vom beweglichen Griffteil auf das Kraftübertragungselement demnach so ausgebildet, dass das Kraftübertragungselement, das wie bereits erwähnt möglichst nur eine axiale Bewegung ausführen sollte, mit einem ebenfalls rein axial beweglichen Schieber verbunden, der passgenau in einem Gleitlager geführt ist. Anstatt wie bei dem aus dem Dokument DE-AS 1 055 751 bekannten Handgriff das bewegliche Griffteil unmittelbar mit dem Schieber zu verbinden, ist bei der erfindungsgemäßen Zange vorgesehen, dass der Schieber über einen Gelenkhebel mit dem beweglichen Griffteil verbunden ist, wobei der Gelenkhebel mit einem Ende am Schieber und mit einem anderen Ende am beweglichen Griffteil angelenkt ist. Der Gelenkhebel dient dem Ausgleich des Niveauunterschiedes zwischen dem Anlenkungspunkt am beweglichen Griffteil und dem Anlenkungspunkt am Schieber. Bei der Erprobung der erfindungsgemäßen Zange hat sich herausgestellt, dass der Gang der Kraftübertragungsmechanik spielfrei ist.

Erfindungsgemäß ist der Schieber in Bezug auf das Kraftübertragungselement parallel versetzt angeordnet und weist einen seitlich abstehenden Mitnehmer auf, an dem das Kraftübertragungselement festgelegt ist.

Diese Ausgestaltung ist insbesondere von Vorteil, wenn die Griffanordnung der Handhabe der Zange scherengriffartig ausgebildet ist. In diesem Fall kann mit der parallel versetzten Anordnung des Schiebers in Bezug auf das Kraftübertragungselement vermieden werden, dass der Gelenkhebel mit dem Schieber einen zu spitzen Winkel bildet. Der Winkel zwischen dem Schieber und dem Gelenkhebel kann vielmehr stumpf gewählt werden, beispielsweise über 130°, vorzuasweise über 150°, wodurch der leichte und spielfreie Gang der Mechanik verbessert wird.

In einer bevorzugten Ausgestaltung weist die Handhabe ein unbewegliches Gehäuseteil auf, das eine einseitig offene Ausnehmung aufweist, in die ein Ende des beweglichen Griffteils eingreift und in der dieses Ende um die Schwenkachse verschwenkbar befestigt ist, und das Gleitlager für den Schieber verschließt die Ausnehmung im Wesentlichen.

Bei der aus dem Dokument DE 38 02 651 C2 bekannten medizinischen Zange ist zwar auch das gelenkige Zwischenstück in der Ausnehmung des Gehäuseteils angeordnet, jedoch verschließt bei dieser bekannten Zange das gelenkige Zwischenstück die Ausnehmung nur unzureichend, da das gelenkige Zwischenstück ja eine Bewegung beim Bewegen des beweglichen Griffteils ausführen muss. Demgegenüber ist das Gleitlager der erfindungsgemäßen Zange ein unbewegliches Teil und kann demnach hinsichtlich seiner Geometrie so ausgebildet werden, dass es die Ausnehmung zumindest im Wesentlichen verschließt. Ein Vorteil, der damit erreicht wird, ist, dass weniger Verunreinigungen in die Ausnehmung des Gehäuseteils eindringen können.

In einer weiteren bevorzugten Ausgestaltung ist das Gleitlager als separates Bauteil ausgeführt.

Ein Vorteil dieser Maßnahme ist die einfachere Herstellbarkeit der erfindungsgemäßen Zange, weil die maschinelle Bearbeitung des Gleitlagers separat von der mechanischen Bearbeitung beispielsweise des zuvor genannten Gehäuseteils erfolgen kann.

In einer weiteren bevorzugten Ausgestaltung ist das Gleitlager hinsichtlich seiner Form so angepasst, dass es bündig mit dem Rand der Ausnehmung des Gehäuseteils abschließt.

Hierbei ist von Vorteil, dass das Gehäuseteil allseitig im Wesentlichen eine von Vorsprüngen freie glatte Oberfläche aufweist, was auch das ästhetische Erscheinungsbild der erfindungsgemäßen Zange wesentlich verbessert.

In einer weiteren bevorzugten Ausgestaltung weist/weisen der Schieber und/oder das Gleitlager eine Länge auf, die größer ist als der maximale Bewegungsweg des Schiebers in dem Gleitlager.

Hierbei ist von Vorteil, dass die exakte lineare Führung des Schiebers in dem Gleitlager weiter verbessert ist, insbesondere wird ein Verkippen und somit Verkanten des Schiebers im Gleitlager vermieden.

In einer weiteren bevorzugten Ausgestaltung ist der Gelenkhebel mittels jeweils einer Niet an dem Schieber und dem beweglichen Griffteil gelenkig festgelegt.

Die Verbindung des Gelenkhebels mittels Nieten am Schieber bzw. am beweglichen Griffteil trägt vorteilhafterweise zusätzlich zur Spielfreiheit des Gangs der Kraftübertragungsmechanik bei.

In einer weiteren bevorzugten Ausgestaltung ist der Gelenkhebel mit dem einen Ende zwischen zwei Schenkeln eines Gabelabschnitts des beweglichen Griffteils und mit dem anderen Ende zwischen zwei Schenkeln eines Gabelabschnitts des Schiebers angelenkt.

Auch diese Maßnahme trägt vorteilhafterweise zur weiteren Verbesserung der spielfreiheit des Gangs der Kraftübertragungsmechanik bei, weil der Gelenkhebel, beispielsweise ein Plättchen, zwischen den jeweiligen Schenkeln der Gabelabschnitte verkippfrei eingefasst werden kann.

In einer weiteren bevorzugten Ausgestaltung weist die Handhabe ein unbewegliches Griffteil auf, wobei das bewegliche und das unbewegliche Griffteil im Wesentlichen quer zur Längsachse des Schafts abstehen und zusammen eine Scherengriffanordnung bilden.

Diese von Ärzten bevorzugte Griffanordnung wird im Unterschied zu dem aus dem Dokument DE-AS 1 055 751 bekannten Handgriff erst durch die erfindungsgemäße Kraftübertragungsmechanik ermöglicht.

Weitere vorteile und Merkmale ergeben sich aus der nachfolgenden Beschreibung und der beigefügten Zeichnung.

Ein Ausführungsbeispiel ist in der Zeichnung dargestellt und wird mit Bezug auf diese hiernach näher beschrieben. Es zeigen:
- Fig. 1: eine medizinische Zange in Seitenansicht;
- Fig. 2: die Handhabe der Zange in Fig. 1 in Alleinstellung in vergrößertem Maßstab und teilweise im Schnitt, in einer ersten Betriebsstellung;
- Fig. 3: die Handhabe in Fig. 2 in einer zweiten Betriebsstellung; und
- Fig. 4: eine isolierte Darstellung der Kraftübertragungsmechanik der Handhabe in Fig. 2 und 3 der Zange in Fig. 1.1.

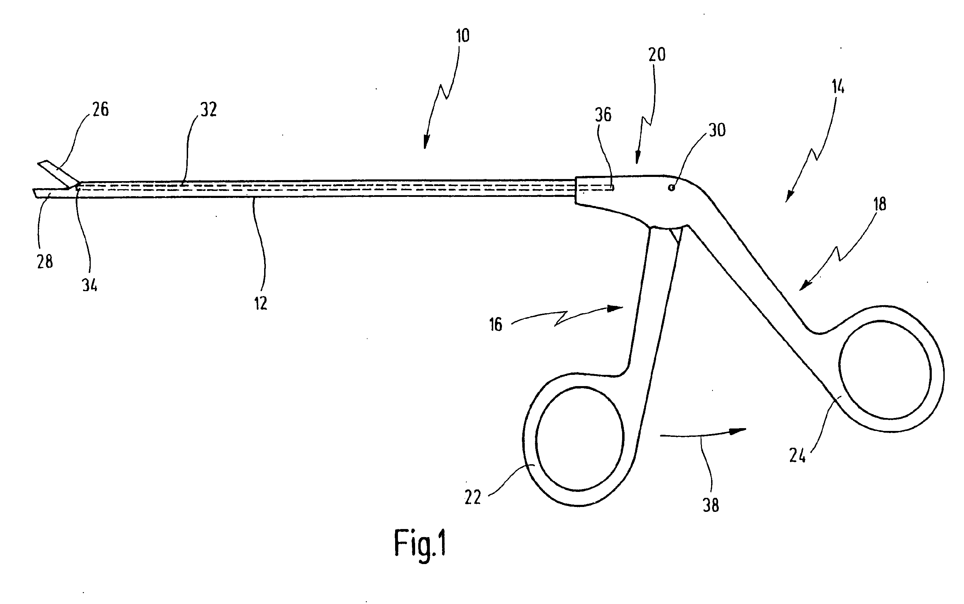

In Fig. 1 ist eine mit dem allgemeinen Bezugszeichen 10 versehene medizinische Zange dargestellt. Die Zange 10 wird im Rahmen chirurgischer Eingriffe am menschlichen oder tierischen Körper, insbesondere in der Endoskop-gestützten minimal-invasiven Chirurgie, verwendet.

Die Zange 10 weist einen lang erstreckten Schaft 12 auf, der einen geringen Durchmesser aufweist, so dass der Schaft 12 durch eine schmale Inzision in den Körper eines Patienten eingeführt werden kann.

Am proximalen Ende des Schafts 12 weist die Zange 10 eine Handhabe 14 auf. Die Handhabe 14 weist ein bewegliches Griffteil 16 und ein unbewegliches Griffteil 18 auf. Des Weiteren weist die Handhabe 14 ein Gehäuseteil 20 auf, das mit dem unbeweglichen Griffteil 18 vorzugsweise einstückig verbunden ist.

Das bewegliche Griffteil 16 weist einen Fingerring 22 zum Durchstecken des Zeige- und/oder Mittelfingers auf, und das unbewegliche Griffteil 18 weist ebenso einen Fingerring 24 zum Durchstecken des Daumens auf.

Am distalen Ende des Schafts 12 weist die Zange 10 ein bewegliches Werkzeug 26 sowie ein unbewegliches Werkzeug 28 auf. Das Werkzeug 28 kann jedoch ebenso wie das Werkzeug 26 beweglich sein.

Die Werkzeuge 26 und 28 können je nach dem Verwendungszweck der Zange 10 schneidend oder fassend zusammenwirken.

Das bewegliche Griffteil 16 ist um eine feststehende Schwenkachse 30 verschwenkbar am Gehäuseteil 20 der Handhabe 14 befestigt. Die Verschwenkung des beweglichen Griffteils 16 dient dem Bewegen des beweglichen Werkzeugs 26. Um die Kraft vom beweglichen Griffteil 16 auf das bewegliche Werkzeug 26 zu übertragen, ist ein Kraftübertragungselement 32 vorhanden, das in Fig. 1 mit unterbrochenen Linien dargestellt ist. Das Kraftübertragungselement 32, beispielsweise ein Zug- und Schubdraht oder -stab, verläuft in Richtung der Längsachse des Schafts 12 und ist relativ zu diesem axial beweglich. In dem gezeigten Ausführungsbeispiel ist der Schaft 12 als Rohr ausgebildet, und das Kraftübertragungselement 32 verläuft entsprechend im Inneren des Schafts 12.

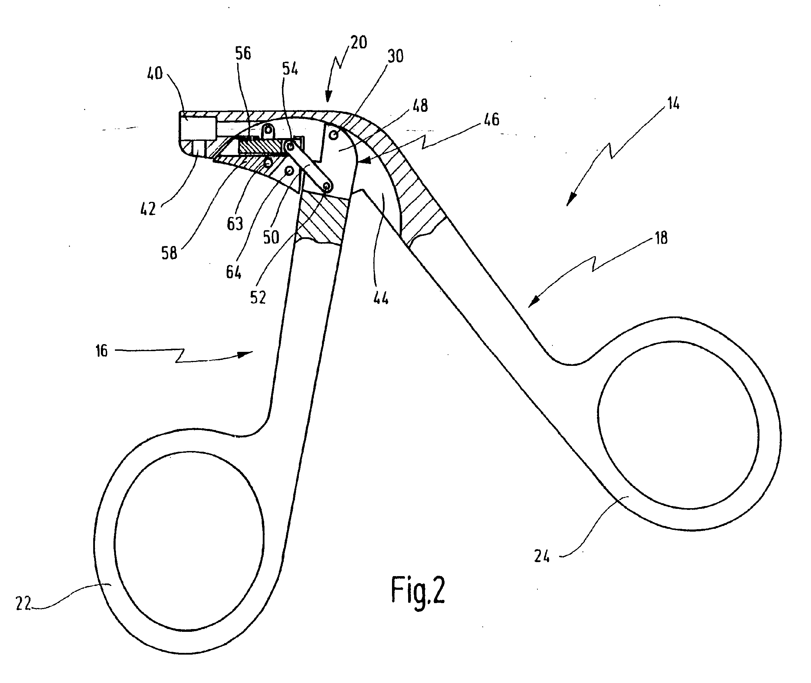

Mit einem Ende 34 steht das Kraftübertragungselement 32 mit dem beweglichen Werkzeug 26 in Wirkverbindung, und mit einem anderen Ende 36 steht das Kraftübertragungselement 32 mit dem beweglichen Griffteil 16 in Wirkverbindung, wie hiernach mit Bezug auf Fig. 2 bis 4 noch näher beschrieben wird.

Das Ende 34 des Kraftübertragungselements 32 ist mit dem beweglichen Werkzeug 26 beispielsweise über eine Gelenkhebelanordnung (nicht dargestellt) verbunden.

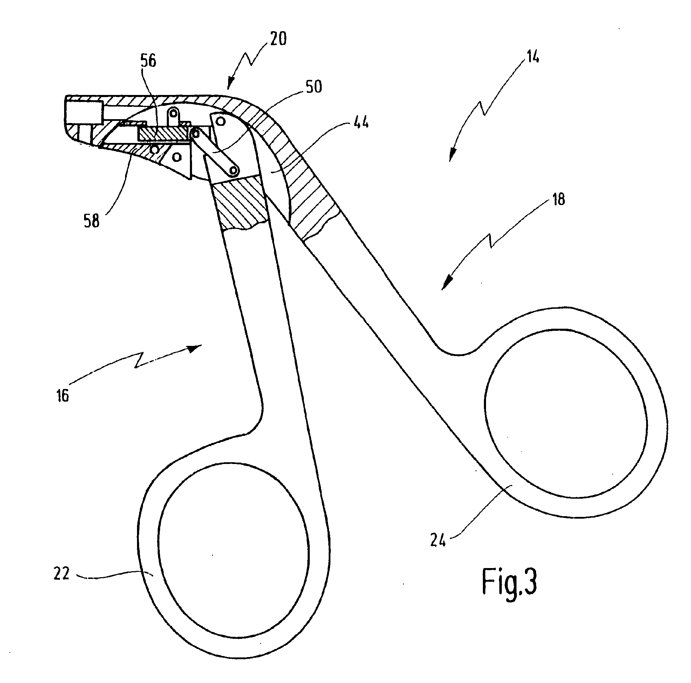

In Fig. 1 ist das bewegliche Werkzeug 26 in Bezug auf das unbewegliche Werkzeug 28 in seiner Offenstellung dargestellt, in der das bewegliche Griffteil 16 von dem unbeweglichen Griffteil 18 maximal beabstandet ist. Durch Verschwenken des beweglichen Griffteils 16 in Richtung eines Pfeiles 38 um die Schwenkachse 30 in Richtung auf das unbewegliche Griffteil 18 wird das bewegliche Werkzeug durch Vermittlung des Kraftübertragungselements 32 zu dem unbeweglichen Werkzeug 28 hin bis in seine Schließstellung bewegt. In der Schließstellung der Werkzeuge 26 und 28 sind die Griffteile 16 und 18 einander angenähert, ohne jedoch aneinander anzuliegen, wie in Fig. 3 dargestellt ist, die die maximal angenäherte Stellung der Griffteile 16 und 18 zeigt.

Die Griffteile 16 und 18 sind so ausgestaltet, dass sie vom Schaft 12 im Wesentlichen quer zur Längsachse des Schafts 12 abstehen und zusammen eine Scherengriffanordnung bilden.

Mit Bezug auf Fig. 2 bis 4 werden nun weitere Einzelheiten insbesondere der Kraftübertragungsmechanik vom beweglichen Griffteil 16 auf das Kraftübertragungselement 32 beschrieben.

Gemäß Fig. 2 weist das Gehäuseteil 20 der Handhabe 14 an seinem distalen Ende eine Bohrung 40 zur Aufnahme des proximalen Endes des Schafts 12 auf. Über eine quer zur Bohrung 40 verlaufende weitere Bohrung 42 wird eine nicht näher dargestellte Madenschraube zum Festklemmen des proximalen Endes des Schafts 12 in der Bohrung 40 eingeschraubt.

Das Gehäuseteil 20, das einstückig mit dem unbeweglichen Griffteil 18 verbunden ist, weist eine einseitig offene Ausnehmung 44 auf, die etwa halbmondförmig ausgebildet ist. Die Ausnehmung 44 ist einseitig, und zwar auf ihrer dem beweglichen Griffteil 16 zugewandten Seite offen.

In diese Ausnehmung ragt ein handhabungsabgewandtes Ende 46 des beweglichen Griffteils 16 hinein, das an der feststehenden Schwenkachse 30, die beispielsweise durch einen Stift gebildet ist, festgelegt ist. Das Ende 46 des beweglichen Griffteils 16 ist als Gabelabschnitt ausgebildet, von dem in Fig. 2 nur ein Schenkel 48 zu sehen ist.

In einem Abstand zur Schwenkachse 30 ist ein Gelenkhebel 50 mit einem Ende an einem Anlenkungspunkt 52 an dem beweglichen Griffteil 16 angelenkt. Der Anlenkungspunkt 52 liegt in Bezug auf die Schwenkachse 30 vom handhabungsseitigen Ende (Fingerring 22) aus gesehen vor der Schwenkachse 30.

Am Anlenkungspunkt 52 ist der Gelenkhebel 50 mittels einer Niet am beweglichen Griffteil 16 festgelegt. Der Gelenkhebel 50 greift dabei in den Gabelabschnitt des beweglichen Griffteils 16 ein, von dem wie bereits erwähnt in Fig. 2 nur der Schenkel 48 zu sehen ist.

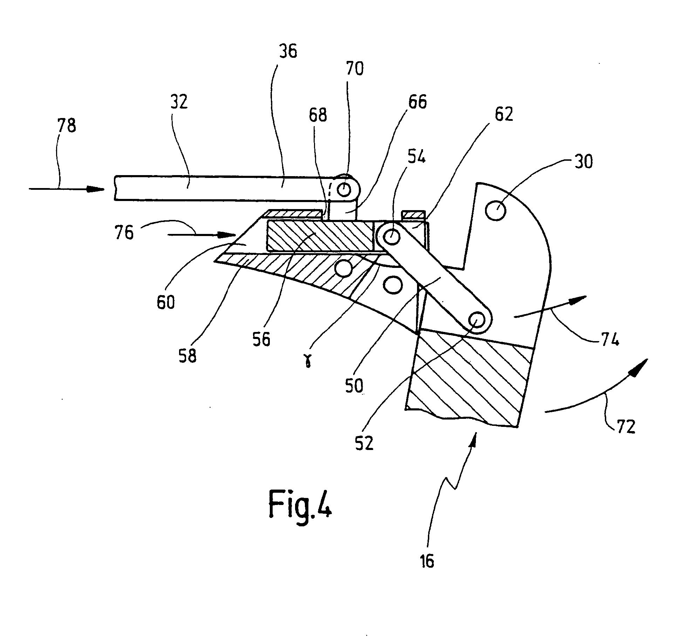

Wie am deutlichsten aus Fig. 4 hervorgeht, ist der Gelenkhebel 50 mit seinem anderen Ende an einem Anlenkungspunkt 54 an einem Schieber 56 angelenkt, der passgenau in einem Gleitlager 58 geführt ist, wobei der Schieber 56 ausschließlich linear in Richtung des Kraftübertragungselements 32 verschieblich ist. Das Gleitlager 58 weist eine entsprechende passgenaue Bohrung 60 auf, die beispielsweise eine H7-Bohrung, also eine mit sehr geringen Toleranzen gefertigte Bohrung, ist. Der Schieber 56 ist zylindrisch ausgebildet und ebenfalls mit geringen Toleranzen gefertigt. Der Schieber 56 ist in der Bohrung 60 des Gleitlagers 58 ohne radiales Spiel gelagert.

An seinem proximalen Ende weist der Schieber 56 im Bereich des Anlenkungspunktes 54 einen Gabelabschnitt auf, von dem in Fig. 4 nur ein Schenkel 62 zu sehen ist.

Wieder mit Bezug auf Fig. 2 ist das Gleitlager 58, das ein separates Bauteil ist, in die Ausnehmung 44 des Gehäuseteils 20 eingesetzt und mittels zweier passgenauer Stifte 63 und 64 am Gehäuseteil 20 unbeweglich festgelegt.

Der Anlenkungspunkt 54 des Gelenkhebels 50 an dem Schieber 56 wird ebenfalls spielfrei durch eine Niet gebildet.

Das Gleitlager 58 ist hinsichtlich seiner Außenkontur so angepasst, dass es die Ausnehmung 44 distalseitig des beweglichen Griffteils 16 im Wesentlichen verschließt und dabei bündig mit dem Rand der Ausnehmung 44 des Gehäuseteils 20 abschließt. Somit ist die Offenseite der Ausnehmung 44 bis auf kleine offene Bereiche, die die Beweglichkeit des beweglichen Griffteils 16 in der Ausnehmung 44 gewährleisten, verschlossen.

Fig. 2 zeigt den Schieber 56 in seiner maximal distalen Position, die der Offenstellung der Werkzeuge 26, 28 bzw. der maximal beabstandeten Stellung des beweglichen Griffteils 16 vom unbeweglichen Griffteil 18 entspricht, und Fig. 3 zeigt den Schieber 56 in der maximal proximalen Stellung, die der Schließstellung der Werkzeuge 26, 28 bzw. der maximal angenäherten Position des unbeweglichen Griffteils 16 zum unbeweglichen Griffteil 18 entspricht.

Wie insbesondere aus Fig. 4 hervorgeht, ist der Schieber 56 bezüglich des Kraftübertragungselements 32 parallel versetzt angeordnet und weist einen seitlich abstehenden Mitnehmer 66 auf, der mit dem Schieber 56 fest verbunden ist. Der Mitnehmer 66 ragt aus einer Öffnung im Gleitlager 58 hervor, wobei der Mitnehmer 66 im Zusammenwirken mit den Rändern der Öffnung 68 im Gleitlager 58 jeweils einen Anschlag für die maximal distale und die maximal proximale Position des Schiebers 56 bildet. Auf diese Weise ist der Schieber 56 auch in dem Gleitlager 58 unverlierbar gehalten.

Der maximale Bewegungsweg des Schiebers 56 in dem Gleitlager 58 ist somit durch die axiale Weite der Öffnung 68 in dem Gleitlager 58 sowie durch die axiale Weite des Mitnehmerelements 66 bestimmt.

Die Länge des Schiebers 56 und die Länge des Gleitlagers 58 bzw. der Bohrung 60 ist größer gewählt als der maximale Bewegungsweg des Schiebers 56 in dem Gleitlager 58.

Das Kraftübertragungselement 32 ist mit seinem Ende 36 an einem Anlenkungspunkt 70 an dem Mitnehmerelement 66 festgelegt.

Der Gelenkhebel 50 bildet mit dem Schieber 56 einen stumpfen Winkel γ (vgl. Fig. 4), was insbesondere durch die parallel versetzte Anordnung zwischen dem Schieber 56 und dem Kraftübertragungselement 32 ermöglicht wird.

Die Funktionsweise der Kraftübertragungsmechanik vom beweglichen Griffteil 16 auf das Kraftübertragungselement 32 wird nachfolgend mit Bezug auf Fig. 4 in Verbindung mit Fig. 2 und 3 beschrieben.

Fig. 4 zeigt das bewegliche Griffteil 16 in derselben Stellung wie in Fig. 2, d.h. in der maximal vom unbeweglichen Griffteil 18 abgespreizten Offenstellung.

Wird nun ausgehend von dieser Stellung das bewegliche Griffteil 16 um die feststehende Schwenkachse 30 gemäß einem Pfeil 72 verschwenkt, führt der Anlenkungspunkt 52 des Gelenkhebels 50 ebenfalls eine Schwenkbewegung in Richtung eines Pfeiles 74 aus. Durch diese Bewegung übt der Gelenkhebel 50 einen Zug auf den Schieber 56 aus, wodurch der Schieber 56 in Richtung eines Pfeiles 76 zurückgezogen wird. Auf Grund der Anlenkung des Gelenkhebels 50 sowohl am beweglichen Griffteil 16 als auch am Schieber 56 und dessen passgenauer Führung in dem Gleitlager 58 wirken auf den Schieber 56 im Wesentlichen keine Querkräfte, so dass dieser eine leichtgängige rein lineare Bewegung in der Bohrung 60 des Gleitlagers 58 ausführt. Über den Mitnehmer 66 wird nunmehr das Kraftübertragungselement 32 ebenfalls rein axial in Richtung eines Pfeiles 78 nach proximal gezogen (in Fig. 2 und 3 ist das Kraftübertragungselement 32 nicht eingezeichnet).

Die umgekehrte Bewegung des beweglichen Griffteils 16 aus der in Fig. 3 dargestellten Schließstellung in die in Fig. 2 dargestellte Offenstellung führt entsprechend zu einer axialen Bewegung des Kraftübertragungselements 32 nach distal.

## Patentansprüche

1. Medizinische Zange, mit einem Schaft (12), einer eine scherengriffartig ausgebildete Griffanordnung aufweisende Handhabe (14) am proximalen Ende des Schafts (12), die zumindest ein im Wesentlichen quer zur Längsachse des Schafts (12) abstehendes bewegliches Griffteil (16) aufweist, das um eine Schwenkachse (30) verschwenkbar ist, mit zumindest einem beweglichen Werkzeug (26) am distalen Ende des Schafts (12), und mit einer Kraftübertragungsmechanik zwischen dem beweglichen Griffteil (16) und dem beweglichen Werkzeug (26) wobei der Gang der Kraftübertragungsmechanik vom beweglichen Griffteil (16) auf das bewegliche Werkzeug (26) spielfrei ist, die ein Kraftübertragungselement (32) aufweist, das in Richtung der Längsachse des Schafts (12) verläuft und axial beweglich ist und mit einem Ende (34) mit dem zumindest einen beweglichen Werkzeug (26) und mit einem anderen Ende (36) mit dem zumindest einen beweglichen Griffteil (16) in Wirkverbindung steht, wobei das Kraftübertragungselement (32) mit einem linear passgenau in einem Gleitlager (58) geführten Schieber (56) verbunden ist, der in Richtung des Kraftübertragungselements (32) axial beweglich ist, und wobei der Schieber (56) über einen Gelenkhebel (50) mit dem beweglichen Griffteil (16) verbunden ist, der mit einem Ende am Schieber (56) und mit einem anderen Ende am beweglichen Griffteil (16) angelenkt ist, **dadurch gekennzeichnet, dass** der Schieber (56) in bezug auf das Kraftübertragungselement (32) seitlich zur Längsachse des Schafts (12) in Richtung zum beweglichen Griffteil (16) hin parallel versetzt angeordnet ist und einen seitlich abstehenden Mitnehmer (66) aufweist, an dem das Kraftübertragungselement (32) festgelegt ist.

2. Zange nach Anspruch 1, **dadurch gekennzeichnet, dass** die Handhabe (14) ein unbewegliches Gehäuseteil (20) aufweist, das eine einseitig offene Ausnehmung (44) aufweist, in die ein Ende (46) des beweglichen Griffteils (16) eingreift und in der dieses Ende (48) um die Schwenkachse (30) verschwenkbar befestigt ist, und dass das Gleitlager (58) für den Schieber (56) die Ausnehmung (44) im Wesentlichen verschließt.

3. Zange nach Anspruch 1 oder 2, **dadurch gekennzeichnet, dass** das Gleitlager (58) als separates Bauteil ausgeführt ist.

4. Zange nach Anspruch 2 oder 3, **dadurch gekennzeichnet, dass** das Gleitlager (58) hinsichtlich seiner Form so angepasst ist, dass es bündig mit dem Rand der Ausnehmung (44) des Gehäuseteils (20) abschließt.

5. Zange nach einem der Ansprüche 1 bis 4, **dadurch gekennzeichnet, dass** der Schieber (56) und/oder das Gleitlager (58) eine Länge aufweist/aufweisen, die größer ist als der maximale Bewegungsweg des Schiebers (56) in dem Gleitlager (58).

6. Zange nach einem der Ansprüche 1 bis 5, **dadurch gekennzeichnet, dass** der Gelenkhebel (50) mittels jeweils einer Niet an dem Schieber (56) und dem beweglichen Griffteil (16) gelenkig festgelegt ist.

7. Zange nach einem der Ansprüche 1 bis 6, **dadurch gekennzeichnet, dass** der Gelenkhebel (50) mit dem einen Ende zwischen zwei Schenkeln (48) eines Gabelabschnitts des beweglichen Griffteils (16) und/oder mit dem anderen Ende zwischen zwei Schenkeln eines Gabelabschnitts (62) des Schiebers angelenkt ist.

8. Zange nach einem der Ansprüche 1 bis 7, **dadurch gekennzeichnet, dass** die Handhabe (14) ein unbewegliches Griffteil (18) aufweist, wobei das bewegliche und das unbewegliche Griffteil (16, 18) im Wesentlichen quer zur Längsachse des Schafts (12) abstehen und zusammen eine Scherengriffanordnung bilden.

## Claims

1. A medical forceps, comprising a shaft (12), a handle (14) having a grip arrangement configured in scissors-grip fashion at the proximal end of the shaft (12), which handle has at least one movable grip part (16) projecting essentially transversely in relation to the longitudinal axis of the shaft and being pivotable about a pivot axis (30), further comprising at least one movable tool (26) at the distal end of the shaft (12), and a force transmission mechanism between the movable grip part (16) and the movable tool (26), the action of the force transmission mechanism from the movable grip part (16) to the movable tool (26) being free from play, which force transmission mechanism has a force transmission element (32), which runs in the direction of the longitudinal axis of the shaft (12) and is axially movable and with one end (34) is in operative connection with the at least one movable tool (26) and with the other end (36) is in operative connection with the at least one movable grip part (16), wherein the force transmission element (32) is connected to a slide (56) which is guided linearly with an exact fit in a sliding bearing (58) and is axially movable in the direction of the force transmission element (32), and wherein the slide (56) is connected to the movable grip part (16) by means of an articulated lever (50), which is articulated with one end on the slide (56) and with another end on the movable grip part (16), **characterized in that** the slide (56) is arranged, with respect to the force transmission element (32), parallel and with respect to the longitudinal axis of the shaft (12) laterally offset and in direction towards the movable grip part (16), and the slide has a laterally projecting driver (66), on which the force transmission element (32) is fixed.

2. The forceps of Claim 1, **characterized in that** the handle (14) has an immovable housing part (20), which has a recess (44) which is open on one side, in which one end (46) of the movable grip part (16) engages and in which this end (46) is fastened in such a way that it can pivot about the pivot axis (30), and **in that** the sliding bearing (58) for the slide (56) essentially doses the recess (44).

3. The forceps of Claim 1 or 2, **characterized in that** the sliding bearing (58) is formed as a separate component.

4. The forceps of Claim 2 or 3, **characterized in that** the sliding bearing (58) is adapted with regard to its shape in such a way that it finishes flush with the edge of the recess (44) of the housing part (20).

5. The forceps of anyone of Claims 1 through 4, **characterized in that** the slide (56) and/or the sliding beating (58) has/have a length which is greater than the maximum path of movement of the slide (56) in the sliding bearing (58).

6. The forceps of anyone of Claims 1 through 5, **characterized in that** the articulated lever (50) is fixed in an articulated manner on the slide (56) and the movable grip part (16) by means of a rivet in each case.

7. The forceps of anyone of Claims 1 through 6, **characterized in that** the articulated lever (50) is articulated with one end between two legs (48) of a forked portion of the movable grip part (16) and/or with the other end between two legs of a forked portion (62) of the slide.

8. The forceps of anyone of Claims 1 through 7, **characterized in that** the handle (14) has an immovable grip part (18), the movable grip part (16) and the immovable grip part (18) projecting essentially transversely in relation to the longitudinal axis of the shaft (12) and together forming a scissors-grip arrangement.

## Revendications

1. Pince médicale, avec une tige (12), une poignée (14) au niveau de l'extrémité proximale de la tige (12), laquelle est munie d'un système de préhension réalisé sous la forme d'une poignée d'une paire de ciseaux et laquelle comporte au moins un élément de préhension mobile (16), qui est en saille sensiblement perpendiculairement à l'axe longitudinal de la tige (12) et qui est apte à pivoter autour d'un pivot (30), avec au moins un outil (26) mobile au niveau de l'extrémité distale de la tige (12), et avec un mécanisme de transmission de force entre l'élément de préhension mobile (16) et l'outil (26) mobile, le rapport du mécanisme de transmission de force étant sans jeu depuis l'élément de préhension mobile (16) jusqu'à l'outil (26) mobile, ledit mécanisme comportant un élément de transmission de force (32), qui s'étend dans la direction de l'axe longitudinal de la tige (12) et est mobile dans la direction axiale, et qui possède une extrémité (34) en liaison active avec ledit au moins un outil (26) mobile et une autre extrémité (36) en liaison active avec ledit au moins un élément de préhension mobile (16), l'élément de transmission de force (32) étant relié à une coulisse (56), qui est guidée linéairement de manière ajustée dans un palier de glissement (58) et qui est mobile axialement dans la direction de l'élément de transmission de force (32), et ladite coulisse (56) étant reliée à l'élément de préhension mobile (16) par un levier articulé (50), qui est articulé avec une extrémité sur la coulisse (56) et avec une autre extrémité sur l'élément de préhension mobile (16), **caractérisée en ce que** la coulisse (56) est disposée parallèlement à l'élément de transmission de force (32) latéralement par rapport à l'axe longitudinal de la tige (12) de manière décalée dans la direction vers l'élément de préhension mobile (16), et comporte un élément entraîneur (66) en saillie latérale, sur lequel est fixé l'élément de transmission de force (32).

2. Pince selon la revendication 1, **caractérisée en ce que** la poignée (14) comporte une partie formant boîtier (20) immobile, qui possède un évidement (44) ouvert sur un côté, dans lequel s'engage une extrémité (46) de l'élément de préhension mobile (16) et dans lequel ladite extrémité (48) est fixée de manière apte à pivoter autour du pivot (30), et **en ce que** le palier de glissement (58) pour la coulisse (56) obture sensiblement l'évidement (44).

3. Pince selon la revendication 1 ou 2, **caractérisée en ce que** le palier de glissement (58) est réalisé sous forme d'élément séparé.

4. Pince médicale selon la revendication 2 ou 3, **caractérisée en ce que** le palier de glissement (58) est ajusté par sa forme de telle sorte qu'il est situé dans le prolongement à fleur du bord de l'évidement (44) de la partie formant boîtier (20).

5. Pince selon l'une quelconque des revendications 1 à 4, **caractérisée en ce que** la coulisse (56) et/ou le palier de glissement (58) a/ont une longueur supérieure à la trajectoire de déplacement maximale de la coulisse (56) dans le palier de glissement (58).

6. Pince selon l'une quelconque des revendications 1 à 5, **caractérisée en ce que** le levier articulé (50) est fixé de manière articulée sur la coulisse (56) et sur l'élément de préhension mobile (16) respectivement au moyen d'un rivet.

7. Pince selon l'une quelconque des revendications 1 à 6, **caractérisée en ce que** le levier articulé (50) est articulé avec l'une des extrémités entre deux branches (48) d'un tronçon en fourche de l'élément de préhension mobile (16) et/ou avec l'autre extrémité entre deux branches d'un tronçon en fourche (62) de la coulisse.

8. Pince selon l'une quelconque des revendications 1 à 7, **caractérisée en ce que** la poignée (14) comporte un élément de préhension immobile (18), l'élément de préhension mobile (16) et l'élément de préhension immobile (18) étant en saillie sensiblement perpendiculairement à l'axe longitudinal de la tige (12) et formant conjointement un système de préhension d'une paire de ciseaux.
